# EUROPEAN PATENT APPLICATION

(11) **EP 1 538 146 A1**
(43) Date of publication of application: **08.06.2005**
(21) Application number: 03078813.7
(22) Date of filing: 05.12.2003
(51) Int. Cl.: C07D 303/16, C07D 301/32, C08F 220/26, C09D 133/10

(54) **Process to modify non glycidyl functional impurities of acid glycidyl esters to be used in further radical polymerisation**

(71) Applicant: Resolution Research Belgium S.A., 1348 Ottignies Louvain-la-Neuve (BE)
(72) Inventor: Heymans, Denis Marie Charles, 1348 Ottignies Louvain-la-Neuve (BE); Henry, Nathalie, 1348 Ottignies Louvain-la-Neuve (BE); Uytterhoeven, Griet, 1348 Ottignies Louvain-la-Neuve (BE)

(57) **Abstract**

The present invention relates to a process to modify the non-glycidyl functional products occurring in industrially produced glycidyl esters, which are currently used in the synthesis of radical polymerisable acrylic or methacrylic acid derivatives, to be further reacted too resins for coating applications. The process to modify non glycidyl functional impurities in acid glycidyl esters of branched alkane carboxylic acids, by reacting the fraction of non-glycidyl functional products with ethylenically unsaturated anhydride.

Another aspect of the present invention is formed by the cured coating films derived from the hereinbefore defined coating compositions and applied on a shaped substrate, and more in particular on automotives.

## Description

The present invention relates to a process to modify the non-glycidyl functional products occurring in industrially produced glycidyl esters, which are currently used in the synthesis of radical polymerisable acrylic or methacrylic acid derivatives, to be further reacted into resins for coating applications.

The presence of such non-glycidyl functional products in industrially produced glycidyl esters and more in particular, those non glycidyl functional products, containing at least one hydroxyl functional group, has been already identified in EP 0 901 481 and US 6, 136, 991 as compounds which deteriorate the final properties of products which are normally prepared from α-branched alkane glycidyl esters. Said documents disclosed the purification of glycidyl esters of α branched alkane carboxylic acids by a thin film vacuum, short pass distillation apparatus. This purification technology was considered as the only suitable, because glycidyl esters are thermally and chemically reactive molecules and separation of these by-products (non-glycidyl functional products) from the glycidyl esters is not easily accomplished. The purification of such a glycidyl ester was found to have a positive contribution to coating film properties and more specifically to the glass transition temperature (Tg) of the polymer(s) and/or oligomer(s) used as coating binders. Adducts of such purified glycidyl esters appeared to cause an improved weatherability and chemical resistances and lower viscosity (i.e., lower VOC) as notable features.

It will be appreciated that the costs,involved with such a purification of initially obtained glycidyl ester by means of expensive thin film vacuum distillation equipment, will be significant.

Therefore due to economical pressure for lower cost manufacture of substantially pure glycidyl esters of branched carboxylic acids, extensive research has been directed to providing such glycidyl ester in an attractive economically way.

An object of the present invention is therefore to provide adducts of glycidyl esters of α-branched alkane carboxylic acids, to be copolymerised into resins showing attractive coating film properties and a lower cost price.

Another object of the present invention is to provide coating resins, providing attractive coating film properties at a lower cost price.

As result of extensive research and experimentation, it has now surprisingly been found that such desired film properties could be achieved with resins derived from industrially produced glycidyl esters which have been pre-reacted with an anhydride. The anhydrides are preferably derived from alkenyl acids or cycloalkenyl acids or the like, and a mixture thereof.

Accordingly the present invention relates to a process for the modification of non-glycidyl functional impurities in glycidyl esters of α branched alkane carboxylic acids, which comprises reacting a glycidyl ester containing a fraction of non-glycidyl functional products with ethylenically unsaturated anhydride.

In addition the present invention relates to resin compositions, obtainable by a process comprising the conversion of the pretreated glycidyl ester with an ethylenically unsaturated acid into an adduct, followed by radical polymerisation with other ethylenically unsaturated monomers.

The obtained pretreated glycidyl ester will be subsequently adducted with a copolymerisable acid such as acrylic or methacrylic acid to be incorporated by radically compolymerisation with other ethylenycally unsaturated comonomers into a thermosetting resin. More in particular the pretreated glycidyl ester will be converted into adducts with acrylic acid, methacrylic acid or maleic acid, fumaric acid or anhydrides thereof, which are radically copolymerisable with acrylate or methacrylate and other vinyl comonomers. The resin is typically produced by heating the ethylenically unsaturated monomers in presence of radical initiator.

The resin compositions are specially adapted for use as a top coat on metal substrate. The said resin is curable with a curing agent, which reacts with the hydroxyl functional groups.

More particularly this invention relates to the reaction of hydroxy functional impurities contained in industrially produced glycidyl ester, and an anhydride and more preferably with acrylic or methacrylic, maleic, fumaric, citraconic, itaconic, tetrahydrophathalic anhydride, and a mixture thereof.

The anhydride concentration is adjusted in an 1:1 to 0.2:1 and preferably from 0.9/1 to 0.4/1 ratio between the measurable hydroxyl content of such products and the anhydride functionality.

Mixing the anhydride with the un-purified glydidyl ester is carried out in a temperature range at which the reaction anhydride-hydroxy is promoted (over acid-epoxy reaction) a range from 40°C to a maximum of 180°C, preferably between 60°C and 160°C and most preferably between 80°C and 160°C. Alternatively a mixture of the anhydride and the un-purified glycidyl ester can be heated from 80°C to 180°C gradually over a period of 30 minutes to 480 minutes, and preferably over a period of 40 to 240 minutes and most preferably over 60 to 120 minutes.

Any un-purified starting alkyl glycidyl esters can be used for the purpose of this invention. Preferably starting alkyl glycidyl esters are used, which correspond to the general formula (I), 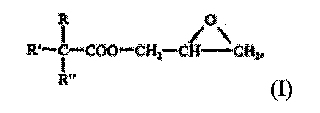 in which R,R',R" are independently hydrogen or aliphatic alkyl radicals of 1 to 10 carbon atoms, while the total number of carbon atoms of the three radicals (R+R'+R")being between 1 to 20, preferably between 2 to 13. These alkyl glycidyl esters are derived for example from 1-methylpropionic acid, 1-dimethyl-propionic acid (pivalic acid), iso-butyric acid, 1-methyl-propionic acid, 1-methyl-butyric acid, 1-dimethyl-butyric acid, VERSATIC acid, neodecanoic glycidyl ester and other glycidyl ester of alkanoic acids and mixture thereof and the like. The most preferred glycidyl esters are glycidyl esters containing in the acid moety from 9 to 13 carbon atoms and most preferred is CARDURA E10P glycidyl ester, which is derived from VERSATIC 10 carboxylic acid (CARDURA & VERSATIC are trademarks from Resolution Performance Products). The said glycidyl esters are obtainable by well established manufacturing processes.

A catalyst for the acid-epoxy reaction can be added in the reactor during the second step after the reaction of the non-glycidyl functional impurities with the anhydride. The catalyst which can be used in the present invention is not especially limited. However, as examples, there can be cited onium salts, heterocyclic amines, inorganic acid, Lewis acids, and organic acid with a pKa ranging -1 to 3, and the like. The preferred catalysts are the organic acids with a pKa ranging from -0.5 to 2,8 and as example paratoluenesulfonic acid, chloro acetic acid, dichloro acetic acid, trichloro acetic acid, trifluoro acetic acid, dimethylol propionic acid and the like and a mixture thereof.

The addition and the reaction time are usually being from 3 to 8 hours, and preferably 5 to 6 hours.

The radical copolymerisation reaction of adducts of glycidyl esters and other ethylenically unsaturated comonomers is carried out at a temperature between 120°C and 200°C, preferably from 130°C to 180°C and more preferably from 140°C to 170°C.

Suitable polymerization initiators to be used for free radical copolymerization include any of the conventional free radical forming compounds, individually or in a mixture. Examples of such compounds are aliphatic azo compounds, diacyl peroxides, peroxy-dicarbonates, alkyl per-esters, alkyl hydroperoxides, per ketals, dialkyl peroxides or ketone peroxides.

Dialkyl peroxides, such as di-tert-butyl peroxide, di-tert-amyl peroxide, and alkyl per esters such as tert-butyl peroxy-2-ethylhexanoate or tert-amyl peroxy-2-ethylhexanoate are preferred.

The concentration of initiator is used in the range of from 1 to 8 wt% and preferably of from 2 to 6 wt%, based on the total mass of the co-monomers.

The ethylenically unsaturated co-monomer may be chosen from aromatic vinyl monomers, preferably styrene also, including substituted styrene such as alpha-substituted vinyl aromatic compounds including vinyl toluene, alpha-methylstyrene and the like. Beta substituted styrene compounds can also be used, such as stylbene, beta-methylstylbene, and the like. Another class of suitable vinyl monomers are alpha-olefins with a carbon chain length of more than 10 and preferably those with 10 to 25 carbon atoms.

Vinyl ethers which contain alkyl, aryl or cycloaliphatic groups with a carbon chain length 5 to 15 and preferably from 5 to 10, can be also used as comonomers.

Vinyl esters such as vinyl acetate, benzoate and Versatate (e.g. VEOVA 10 or VEOVA 9 vinyl esters (VEOVA is a trademark ex RPP) and the like are suitable co-monomers to be used in the process of this invention.

Alkyl acrylate and/or an alkyl methacrylate, contains an alkyl group having from 1 to 12 carbon atoms, and preferably from 1 to 10 carbon atoms. Particularly suitable monomers of the group are selected from methyl-, ethyl-, propyl-, isopropyl, n-butyl, iso-butyl, isobornyl esters of acrylic acid or methacrylic acid.

More preferred are methyl acrylate or methyl methacrylate, ethyl acrylate, ethyl methacrylate, n-butyl acrylate or n-butyl methacrylate.

Other alkyl unsaturated monomers, such as symmetric or assymetric unsaturated diesters, diacid or anhydride e.g. alkyl maleate, alkyl fumarate, maleic acid or anhydride and fumaric acid and the like could be part of the co-monomer mixture.

Suitable cross-linking agents to be applied in the coating compositions of the present invention can be selected in general from blocked isocyanate groups containing compounds. Polyisocyanate may be aliphatic, cycloaliphatic, or heterocyclic and may be substituted or unsubstituted.

For curing above 100°C the polyisocyanate is preferably a fully-capped (blocked) polyisocyanate with substantially no free isocyanate groups. The polyisocyanate is preferably aliphatic. Diisocyanates are preferred, although higher polyisocyanates can be used in place of or in combination with diisocyanates. Suitable isocyanates for curing at room temperature are described in "Polyurethanes for coating" (Ed. By Dr. Ulrich Zorll,author Dr. Manfred Bock; pages 109 to 150)which is included herein by reference.

Other types of hereinbefore specified cross-linking agents can be formaldehyde adducts derived from urea, melamine and benzoguanamine, or the etherified formaldehyde-amine adducts. Preferred cross-linking agents for the formaldehyde adducts are the melamine-formaldehyde adducts partially or completely etherified with aliphatic alcohols having 1 to 4 carbon atoms. Examples of such commercially available curing agents are: MAPRENAL MF900, VMF3296 and CYMEL 303, 327 and 1158 curing agents (MAPRENAL, VMF and

CYMEL are trademarks).

The cross-linking reaction can be catalyzed by addition of an organometallic compound, such as tin compounds, and if desired tertiary amines, preferably diethyl ethanol amine. Examples of appropriate tin compounds are di-butyl tin dilaurate, di-butyl tin acetate and di-butyl oxo-tin.

It will be appreciated that another aspect of the present invention is formed by the hydroxyl containing resin composition to be used in coating compositions, which provide a fast drying time of the said coating, while maintaining other relevant physical properties such as hardness, MEK resistance, VOC flexibility, scratch resistant and acid resistant coating films after curing.

Another aspect of the present invention is formed by the curable coating films derived from the hereinbefore defined coating compositions and applied on a shaped substrate, and more in particular on automotives.

### Test description and analytical methods:

Acid value: according to ISO 3682-96

Drying test:
Dust free time:
   The dust free time is measured by falling from a distance of 5 cm a piece of cotton wool onto the top of the coated panel. When the panel is turned upside down and the cotton falls off, the coating is assumed to be dust free. The time is noted.
Tack free time:
   The tack free time is the drying time (at room temperature)that is needed before a Koenig hardness of 20s is achieved. The pendulum hardness test ISO 1522-73 is used.

### Molecular weight determination:

General: General information on liquid exclusion chromatography can be obtained from ASTM D3016; standard practice for use of liquid exclusion chromatography terms and relationships.

*Scope:* This method describes the determination of the molecular size distribution characteristics of acrylic copolymer resins.

*For the correlation, the following standars are used:* polystyrene standards, covering the relative molecular mass range from about 5000 to 3000000 the following Toya Soda standars are recommended: A-5000, F-1, F-2, F-4, F-10, F-20, F-40, F-80, F128, F-288 and 1-phenylhexane.

### Method conditions:

- PL (Polymer Laboratories) columns were used.
- Short column set : small pre-column and 3 columns of 30 cm PL-gel 5 micron: mixed C + 2X mixed D
- Column oven at 40°C
- Mobile phase: THF at 0.8ml/min
- UV- and RI detector

Samples were dissolved in THF. Mn, Mw, Peak and Mwd are quoted.
Solids content: according to ASTM D2369-92
Volatile organic content (VOC): according to ASTM D 3960-96
Pendulum damping test (Koenig hardness): according to

### ISO 1522-73(E) or DIN 53 157

### Resistance of coatings against acid Method summary:

The acid resistance is determined by exposing the film to droplets of 0.6N sulphuric acid for 30, 60 and 90 minutes at 50°C and assessing the resulting damage to the film. Minimum score (per spot) is 0 (very poor), maximum score is 10 (not affected), and the three scores are then added together. The acid resistance was measured on coatings cured at room temperature and cured at 60°C for 30 minutes.

### MEK resistance (MEK) Summary of the method:

A piece of cotton wool, soaked with MEK, is rubbed over the coating (using a pressure of around 2 kg). If the coating surface has been rubbed one hundred times up and down without showing any damage, the coating is said to be fully cured. If, after x "double MEK rubs", the coating has been destroyed on a full line going from the top to the bottom of the panel, the coating is said to fail after x "double MEK rubs". The MEK resistance was measured on coatings cured at room temperature and cured at 60°C for 30 minutes. Measurement of the resistance to cracking-rapid deformation (Impact test /Kugelschlagprufung/ Falling Weight test): according to ISO 6272-93(E).

### Measurement of resistance to cracking-gradual deformation (Erichsen Slow Penetration test or Cupping test)

According to: ISO 1520-73(E)
Viscosity of Newtonian systems using a Brookfield viscometer: ISO 2555-89.

The invention is illustrated in more detail in the following examples, however without restricting its scope to these specific embodiments.

### EXAMPLES

### Example 1

Equipment: Glass reactor equipped with an anchor stirrer, reflux condenser and nitrogen flush.

The reactor was charged with 150 grams of Cardura E10P, 12.5 grams of Xylene and 2.3 grams of methacrylic anhydride. The mixture was heated under agitation to a maximum of 80°C for 1 hour. Subsequently the following copolymerisation manufacturing procedure was applied. The monomer composition is reported in Table 1.

The initial reactor charge (see above description) was heated to the polymerization temperature of 160°C. The monomers-initiator mixture was dosed continuously to the reactor at a rate of 1.37 g/min. After completion of the mixture addition, an additional part of initiator was then fed together with a small amount of the butyl acetate to the reactor in 30 minutes. The reactor was kept at this temperature for 60 more minutes. Finally, the polymer was cooled down, diluted with the remaining butyl acetate and filtered.

**TABLE 1:**

| Resin composition and properties according to the invention. | | |
|---|---|---|
| **Example** | | **1** |
| Initial Reactor Charge | Xylene/Cardura/anhydride | 32.86 |
| Monomer feed | | |
| | Xylene | 8.5 |
| | Hydroxyethyl methacrylate | 15.9 |
| | Styrene | 21.9 |
| | Methylacrylic acid | 10.6 |
| | Methyl methacrylate | 21.2 |
| | Di ter butyl peroxide | 4 |
| Post cook | DTBP | 1 |
| Butyl acetate | | 8 |
| Solids content | | 74.69 |
| (intakes : all expressed in part by weight) | | |

### Comparative examples A-B.

Equipment: Glass reactor equipped with an anchor stirrer, reflux condenser and nitrogen flush.

The reactor was charged with 30 grams of Cardura E10P, 11 grams of Xylene. The polymerization conditions as for example 1 and the composition are given in Table 2.

**TABLE 2:**

| Comparative examples: resin composition and properties. | | | |
|---|---|---|---|
| **Comparative Example** | | **A** | **B** |
| | | **MACE** | **IBMA** |
| Initial Reactor Charge | Cardura E10P | 30 | 22.3 |
| | Xylene | 4 | 4 |
| Monomer feed | | | |
| | Xylene | 7 | 7 |
| | Methacrylic acid | 10.7 | 7.9 |
| | Hydroxyethyl methacrylate | 16 | 20.1 |
| | Styrene | 22 | 30 |
| | Methyl methacrylate | 21.3 | 19.2 |
| | DTBP | 4 | 4 |
| Post cook | DTBP | 1 | 1 |
| Butyl acetate | | 8 | 8 |
| Solids content | | 73.08 | 73.06 |
| (intakes : all expressed in part by weight) | | | |

### Cured coating compositions

Clear lacquers were formulated from the acrylic copolymer resins by the addition of said copolymer resin to an isocyanate curing agent (Desmodur N 3600) in a 1/1 OH/isocyanate ratio and a curing catalyst (Di-butyl tin dilaurate) at a concentration of 0.01%, based on copolymer resin and isocyanate content. Solvent, butyl acetate was added to dilute at an applied viscosity of 100 m Pas. The coating properties of formulations based on resins according to the invention are reported in Table 3 and in Table 4 :for the comparative examples.

**TABLE 3:**

| Coating properties according to the invention | |
|---|---|
| **Formulation** | **1** |
| **Resin of example** | **1** |
| VOC g/l * | 392 |
| Dust free time, minutes | 72 |
| Tack free time, RT, hours | 15 |
| Koening hard, RT, after 4 hrs, s | tacky |
| Koening hard, RT, after 168 hrs, s | 154 |
| MEK resistance, double rubs | >100 |
| Acid resistance | 29 |

| | |
|---|---|
| * of the coating composition as applied at a viscosity of 100 mPas. | |

**TABLE 4:**

| Coating properties of the comparative resins | | |
|---|---|---|
| **Comparative Formulation** | a | b |
| Resin of Comp. example | A | B |
| VOC g/l * | 383 | 373 |
| Dust free time, minutes | 71 | 65 |
| Tack free time, RT, hours | 25 | 15 |
| Koening hard, RT, after 4 hrs, s | tacky | tacky |
| Koening hard, RT, after 168 hrs, s | 105 | 151 |
| MEK resistance, double rubs | >100 | >100 |
| Acid resistance | 29 | 28 |

| | | |
|---|---|---|
| * of the coating composition as applied at a viscosity of 100 mPas. | | |

## Claims

1. A process to modify non glycidyl functional impurities in acid glycidyl esters of branched alkane carboxylic acids, which comprises the reaction product of the fraction of non glycidyl functional products with ethylenically unsaturated anhydride.

2. The process according to claim 1 **characterised in that** the non-glycidyl functional products contains at least one hydroxyl functional group.

3. The process according to claims 1-2 **characterised in that** the ethylenically unsaturated anhydride is derived from alkenyl, cycloalkenyl acids.

4. The process according to claim 1 **characterised in that** the starting glycidyl ester comprises an alkyl glycidyl ester corresponding to the general formula (I), 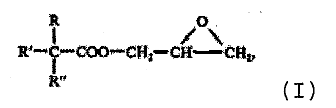 in which R,R',R" are independently hydrogen or aliphatic alkyl radicals of 1 to 10 carbon atoms, the total carbon atoms of the three radicals (R+R'+R")being between 1 to 20, preferably between 2 to 13.

5. The compounds of claim 4 **characterised in that** the glycidyl ester is selected from 1-methylpropionic glycidyl ester, 1-dimethyl-propionic glycidyl ester (pivalic glycidyl ester), iso-butyric glycidyl ester, 1-methyl-butyric glycidyl ester, 1-dimethyl-butyric glycidyl ester, Versatic acid glycidyl ester, neodecanoic glycidyl ester and other glycidyl ester of alkanoic acids and mixture thereof.

6. The process according to claims 1-2 **characterised in that** the ethylenically unsaturated anhydride is acrylic or methacrylic, maleic, fumaric, citraconic,itaconic, tetrahydrophathalic anhydride.

7. Resins composition obtainable by a process according to claims 1-6 followed by further reaction of the glycidyl functional group into an adduct with an ethylenically unsaturated acid monomer and subsequent radical copolymerisation of this adduct with other ethylenically unsaturated comonomers.

8. The resin composition according to claim 7 obtainable by radical polymerisation of the unsaturated hydroxyl-esters with at least one vinyl monomer.

9. A curable coating composition comprising the resin composition of claims 7 and 8 wherein the said copolymer is cross-linked with a curing agent for hydroxyl functionality, applied on a shaped substrate.

10. A cured coating film obtainable by curing the curable coating composition of claim 9.
